# EUROPEAN PATENT APPLICATION

(11) **EP 3 407 065 A1**
(43) Date of publication of application: **28.11.2018**
(21) Application number: 17741470.3
(22) Date of filing: 19.01.2017
(51) Int. Cl.: G01N 33/543, G01N 33/53, G01N 33/72, G01N 33/558

(54) **CHROMATOGRAPH MEDIUM**

(30) Priority: 22.01.2016 JP 2016010795
(71) Applicant: Tanaka Kikinzoku Kogyo K.K., Chiyoda-ku Tokyo 100-6422 (JP)
(72) Inventor: KATO Shinichi, Hiratsuka-shi Kanagawa 254-0076 (JP); MIYATA Aki, Hiratsuka-shi Kanagawa 254-0076 (JP); ITO Daisuke, Hiratsuka-shi Kanagawa 254-0076 (JP)
(74) Representative: Hoffmann Eitle
(86) International application number: PCT/JP2017/001698
(87) International publication number: WO 2017/126593

(57) **Abstract**

An object of the present invention is to provide a chromatographic medium having sufficiently improved storage stability. The present invention relates to a chromatographic medium having a detection part in which a detection substance composed of a protein is fixed, wherein the detection part includes a tri- or higher polysaccharide and a basic amino acid.

## Description

### Technical Field

The present invention relates to a chromatographic medium, an immunochromatographic device, an immunochromatographic kit, and an immunochromatographic analysis method.

### Background Art

Recently, the importance of an immunoassay by immunochromatography which does not require a pretreatment of a specimen as, for example, a simple in vitro diagnostic kit or a portable diagnostic device for detecting an antigen in a sample solution utilizing a specific reactivity of an antibody has been increasing. In particular, a test kit for a pathogen such as a virus or a bacterium is an immunochromatographic device which is familiar and widely used also in ordinary hospitals and clinics.

The simplest structure of the conventional immunochromatographic device is a structure in which a sample addition part, a labeling substance retaining part, a chromatographic medium (chromatographic medium part) having a detection part, and an absorption part for absorbing a liquid having passed through the detection part are mutually connected to each other.

Among the above-mentioned respective parts, in the detection part of the chromatographic medium, a protein such as an antibody which binds to a substance to be detected as a detection target is fixed. This protein is denatured depending on temperature conditions or humidity conditions, resulting in a decrease in detection sensitivity to a detection target, and therefore, there was a problem that the immunochromatographic device cannot be stored stably for a long time. Therefore, a means for enhancing the storage stability by suppressing the denaturation of a protein included in the detection part of the chromatographic medium has been variously studied.

For example, Patent Document 1 discloses an immunochromatographic device having storage stability, in which a sugar or a sugar derivative is used in a solid phase of a detection part or the like of the immunochromatographic device so as to keep the humidity in the device appropriate.

Further, Non-Patent Document 1 discloses an immunochromatographic device, in which each of an amino acid such as glycine or lysine and a sugar such as sucrose or trehalose is included singly in a membrane of a chromatographic medium so as to enhance the storage stability of an antibody and suppress the decrease in the detection sensitivity.

### Related Art Documents

### Patent Document

Patent Document 1: WO 2002/40999

### Non-Patent Document

Non-Patent Document 1: METHODS 22, 53-60 (2000) "Development of Rapid One-Step Immunochromatographic Assay"

### Disclosure of the Invention

### Problems to Be Solved by the Invention

However, it still could not be said that the effect of the prior art as described above is sufficient, and there was still room for improvement. An object of the present invention is to provide a chromatographic medium having sufficiently improved storage stability.

### Means for Solving the Problems

As a result of intensive studies for solving the above-mentioned problems, the present inventors found that in an immunochromatographic device to be used in immunochromatography, by including both a specific sugar and a specific basic amino acid in a detection part of a chromatographic medium, the denaturation of a protein which is a detection target fixed in the detection part is sufficiently suppressed so as to sufficiently enhance the storage stability, and thus, the decrease in the activity of the detection substance can be prevented, and also found that the activity of the detection substance can also be improved, and thus, completed the present invention.

That is, the present invention is as follows.
1. A chromatographic medium which is used in immunochromatography for detecting a substance to be detected in a specimen, comprising: a detection part in which a detection substance composed of a protein is fixed, wherein the detection part includes a tri- or higher polysaccharide and a basic amino acid.
2. The chromatographic medium according to above 1, wherein the polysaccharide included in the detection part is at least one polysaccharide selected from the group consisting of raffinose, nigerotriose, maltotriose, melezitose, maltotriulose, kestose, nystose, nigerotetraose, stachyose, and maltotetraose.
3. The chromatographic medium according to above 1 or 2, wherein the detection part includes the polysaccharide in an amount of 5 to 50 nmol.
4. The chromatographic medium according to any one of above 1 to 3, wherein the detection part includes the basic amino acid in an amount of 5 to 50 nmol.
5. The chromatographic medium according to any one of above 1 to 4, wherein the molar ratio of the polysaccharide to the basic amino acid included in the detection part is from 1:10 to 10:1.
6. The chromatographic medium according to any one of above 1 to 5, wherein the detection part includes at least one basic amino acid selected from the group consisting of arginine, lysine, ornithine, and histidine.
7. The chromatographic medium according to any one of above 1 to 6, wherein the detection part includes lysine.
8. The chromatographic medium according to any one of above 1 to 7, wherein the substance to be detected is a glycoprotein.
9. The chromatographic medium according to above 8, wherein the glycoprotein is HbA1c.
10. An immunochromatographic device, comprising: the chromatographic medium according to any one of above 1 to 9.
11. An immunochromatographic kit, comprising: the immunochromatographic device according to above 10 and a specimen diluent.
12. An immunochromatographic analysis method, wherein the following steps (1) to (4) are sequentially performed using the immunochromatographic kit according to above 11:
   (1) a step of adding a specimen to a sample addition part along with a specimen diluent,
   (2) a step of allowing a labeling substance retained in a labeling substance retaining part to recognize a substance to be detected contained in the specimen,
   (3) a step of developing the specimen and the labeling substance in a chromatographic medium as a mobile phase; and
   (4) a step of detecting the substance to be detected in the developed mobile phase in a detection part.

### Effects of the Invention

According to the present invention, a chromatographic medium, in which the denaturation of a protein which is a detection target fixed in a detection part is sufficiently suppressed, the storage stability is sufficiently high, and the decrease in the activity of a detection substance can be prevented, can be provided. Further, the activity of the detection substance can also be improved.

### Brief Description of the Drawings

[FIG. 1] FIG. 1 is a cross-sectional view for explaining a structure of an immunochromatographic device.
[FIG. 2] FIG. 2 is a graph showing the relative activity of the color development value of a detection part in Examples 1 and 2 and Comparative Examples 1 and 2.
[FIG. 3] FIG. 3 is a graph showing the relative activity of the color development value of a detection part after two days of humidification in Examples 3 to 6 and Comparative Examples 3 to 10.

### Embodiments for Carrying Out the Invention

Hereinafter, embodiments for carrying out the present invention will be described.

Incidentally, in this description, the "fixing" means that an antibody or the like is placed on a carrier such as a film so as not to move, and the "retaining" means that an antibody or the like is placed movably in a carrier such as a film or on the surface thereof.

The chromatographic medium of the present invention has a detection part in which a detection substance composed of a protein is fixed, and is characterized in that the detection part includes a tri- or higher polysaccharide and a basic amino acid.

The detection part is formed on the chromatographic medium. That is, a protein which is the detection substance to bind to a substance to be detected is immobilized on an arbitrary position.

In the chromatographic medium of the present invention, by including a tri- or higher polysaccharide in the detection part, a protein which is the detection substance in the detection part is considered to have moisture resistance, and as a result, the denaturation of the protein is suppressed so as to enhance the storage stability, and thus, the decrease in the activity of the detection substance can be prevented.

Further, by using a tri- or higher saccharide having many hydrogen-bondable hydroxy groups per molecule among the polysaccharides, a higher effect than monosaccharides or disaccharides can be obtained.

The mechanism of action capable of enhancing the storage stability of the detection substance by including a tri- or higher polysaccharide in the detection part of the chromatographic medium of the present invention is not clear, however, it is considered that the tri- or higher polysaccharide maintains the hydrogen bond network of the protein which is the detection substance, and therefore, when bound water is released from the protein which is the detection substance by drying, the destruction of the conformation of the protein is prevented and the denaturation of the protein is prevented so as to enhance the storage stability, and thus, the decrease in the activity can be prevented.

The polysaccharide is not particularly limited as long as it is a tri- or higher polysaccharide. Examples thereof include a trisaccharide or a tetrasaccharide as described below.

Examples of the trisaccharide include raffinose, nigerotriose, maltotriose, melezitose, maltotriulose, and kestose.

Examples of the tetrasaccharide include nystose, nigerotetraose, stachyose, and maltotetraose.

In the present invention, among the polysaccharides, a trisaccharide or a tetrasaccharide is preferred. Above all, it is preferably at least one polysaccharide selected from the group consisting of raffinose, nigerotriose, maltotriose, melezitose, maltotriulose, kestose, nystose, nigerotetraose, stachyose, and maltotetraose. It is more preferably a trisaccharide, and further more preferably raffinose among the trisaccharides.

It is because when the polysaccharide is the above-mentioned saccharide, the inhibition of the reaction between a substance to be detected and a detection substance is suppressed, the hydrogen bond network of the protein which is the detection substance is appropriately maintained so as to increase the storage stability, and thus, the decrease in the activity can be prevented. Many of the tri- or higher polysaccharides are nonreducing sugars, and do not cause functional group transformation to an aldehyde or a ketone having a weak hydrogen bonding ability by hydrolysis, and therefore, a rigid hydrogen bond network can be maintained.

In the detection part of the chromatographic medium of the present invention, among the above-mentioned saccharides, one type may be included, or two or more types may be mixed and included.

The content of the polysaccharide in the detection part is, for example, from 5 to 50 nmol, preferably from 10 to 40 nmol, more preferably from 20 to 30 nmol per chromatographic medium. It is because by setting the content within the above range, the enhancement of the storage stability and the prevention of the decrease in the activity can be sufficiently achieved, and the inhibition of the reaction between a substance to be detected and a detection substance can be further suppressed.

Further, in the chromatographic medium of the present invention, by including a basic amino acid along with the above-mentioned polysaccharide in the detection part, the protein which is the detection substance in the detection part has moisture resistance, and as a result, the denaturation of the protein is suppressed so as to enhance the storage stability, and this, the decrease in the activity of the detection substance can be prevented. Further, because of being a basic amino acid, as compared with the other amino acids, a higher effect can be obtained.

The mechanism of action capable of enhancing the storage stability of the detection substance by including a basic amino acid in the detection part of the chromatographic medium of the present invention is not clear, however, it is considered that the basic amino acid weakly interacts with the protein which is the detection substance so as to suppress the association and aggregation of the proteins, and thus, the decrease in the activity of the detection substance can be prevented.

The type of the basic amino acid is not particularly limited, but examples thereof include arginine, lysine, ornithine, and histidine. From the viewpoint of enhancing the storage stability and further preventing the decrease in the activity, preferably, lysine and arginine are used, and lysine is most suitably used. In the detection part of the chromatographic medium of the present invention, among the above-mentioned basic amino acids, one type may be included, or two or more types may be mixed and included.

The content of the basic amino acid in the detection part is, for example, from 5 to 50 nmol, preferably from 10 to 40 nmol, more preferably from 15 to 30 nmol per chromatographic medium. It is because by setting the content within the above range, the decrease in the activity of the detection substance can be further suppressed.

In the chromatographic medium of the present invention, the detection part includes both a tri- or higher polysaccharide and a basic amino acid, and the storage stability of the detection substance is remarkably improved as compared with the case where the detection part includes each of the components singly.

The mechanism of the action is not clear, however, it is presumed that by including both a tri- or higher polysaccharide and a basic amino acid in the detection part, the polysaccharide forms a hydrogen bond network so as to keep the correct conformation, and also the basic amino acid weakly interacts with the antibody so as to suppress aggregation of the antibodies, and thus, the activity is maintained for a long time.

It is also presumed that in the detection of a substance to be detected by the detection substance, the association between the polysaccharide and the detection substance can be moderately disentangled by the basic amino acid without deteriorating the effect of the polysaccharide on the storage stability, and therefore, the decrease in the reaction activity between the substance to be detected and the detection substance can also be suppressed.

The ratio of the polysaccharide to the basic amino acid to be included in the detection part is generally from 1:10 to 10:1, preferably from 1:4 to 4:1, more preferably from 4:5 to 5:4 in mass (molar) ratio. By setting the ratio within the above range, a synergistic effect obtained by including both the polysaccharide and the basic amino acid becomes more effective.

The protein which is the detection substance to be fixed in the detection part of the chromatographic medium of the present invention is not particularly limited as long as it is a protein which specifically binds to a detection target, and examples thereof include an antibody, an antigen, and a hormone.

Examples of the antibody include a polyclonal antibody or a monoclonal antibody, and a fragment thereof. As the monoclonal antibody and the polyclonal antibody or the fragment thereof, those known and commercially available can be used, or those can be prepared by a known method.

The fixing amount of the antibody is, for example, from 0.1 to 1 µg, preferably from 0.2 to 0.6 µg, more preferably from 0.3 to 0.4 µg per chromatographic medium.

The chromatographic medium of the present invention can be used as a developing part of an immunochromatographic device. The chromatographic medium is an inactive film composed of a microporous material exhibiting a capillary phenomenon. From the viewpoint of having no reactivity with a detection reagent, an immobilization reagent, a substance to be detected, and the like to be used in immunochromatography and also from the viewpoint of enhancing the effect of the present invention, for example, a membrane made of nitrocellulose (hereinafter sometimes referred to as "nitrocellulose membrane") or a membrane made of cellulose acetate (hereinafter sometimes referred to as "cellulose acetate membrane") is preferred, and a nitrocellulose membrane is more preferred. Incidentally, it is also possible to use a cellulose-based membrane, a nylon membrane, and a porous plastic cloth (polyethylene or polypropylene).

The nitrocellulose membrane may be any as long as it includes nitrocellulose as a main component, and a membrane which includes nitrocellulose as a main material such as a pure product or a nitrocellulose mixed product can be used.

In the nitrocellulose membrane, a substance which promotes a capillary phenomenon can also be further included. As the substance, a substance which lowers the surface tension of the film surface to impart hydrophilicity is preferred.

For example, a substance, which has an amphiphilic action such as a variety of synthetic surfactants or alcohols, and which has no effect on the migration of a substance to be detected on the chromatographic medium, and does not affect the development of the color of a marker substance such as colloidal gold is preferred.

The nitrocellulose membrane is porous and exhibits a capillary phenomenon. The index of this capillary phenomenon can be confirmed by measuring a water absorption rate (water absorption time: capillary flow time). The water absorption rate affects the detection sensitivity and the test time.

The form and size of the chromatographic medium represented by a nitrocellulose membrane or a cellulose acetate membrane as described above are not particularly limited, and may be any as long as they are appropriate in terms of actual operation and observation of the results of the reaction.

In addition, in order to prevent the decrease in the analysis accuracy due to nonspecific adsorption, the chromatographic medium of the present invention can be subjected to a blocking treatment by a known method as needed.

In general, in the blocking treatment, a protein such as bovine serum albumin, skim milk, casein, or gelatin is preferably used. After such a blocking treatment, according to need, for example, washing may be performed by using one surfactant or two or more surfactants in combination such as polyethylene glycol sorbitan monolaurate (for example, Tween 20, manufactured by Wako Pure Chemical Industries, Ltd.), polyoxyethylene octyl phenyl ether (for example, Triton X-100, manufactured by Wako Pure Chemical Industries, Ltd.), and sodium dodecyl sulfate (for example, SDS, manufactured by Wako Pure Chemical Industries, Ltd.).

Further, in the detection part in the chromatographic medium of the present invention is formed on the chromatographic medium. That is, the above-mentioned protein which specifically binds to a substance to be detected is immobilized on an arbitrary position.

The specimen containing a substance to be detected is not particularly limited, but examples thereof include biological samples, that is, whole blood, serum, plasma, urine, saliva, sputum, a nasal swab or a pharyngeal swab, spinal fluid, amniotic fluid, nipple discharge fluid, tear, sweat, exudates from the skin, and extracts from tissues, cells, and feces, and in addition thereto, milk, eggs, wheat, beans, beef, pork, chicken, and extracts from foods and the like containing the same.

Specific examples of the substance to be detected include carcinoembryonic antigen (CEA), HER2 protein, prostate specific antigen (PSA), CA19-9, α-fetoprotein (AFP), immunosuppressive acidic protein (IPA), CA15-3, CA125, estrogen receptor, progesterone receptor, fecal occult blood, troponin I, troponin T, CK-MB, CRP, human chorionic gonadotropin (hCG), luteinizing hormone (LH), follicle-stimulating hormone (FSH), syphilis antibody, influenza virus, human hemoglobin, chlamydia antigen, group A β-hemolytic streptococcal antigen, HBs antibody, HBs antigen, rotavirus, adenovirus, albumin, and glycoproteins such as glycated albumin and glycated hemoglobin, however, the substance to be detected is not limited thereto.

Above all, the substance to be detected is preferably a glycoprotein, more preferably glycated hemoglobin, and particularly preferably HbA1c. It is because, an HbAlc antibody should recognize a slight difference between a state where glucose binds to hemoglobin and a state where glucose does not bind to hemoglobin, and the conformation thereof should be strictly maintained, and therefore, in the case where the substance to be detected is HbA1c., the effect of the present invention particularly effectively functions.

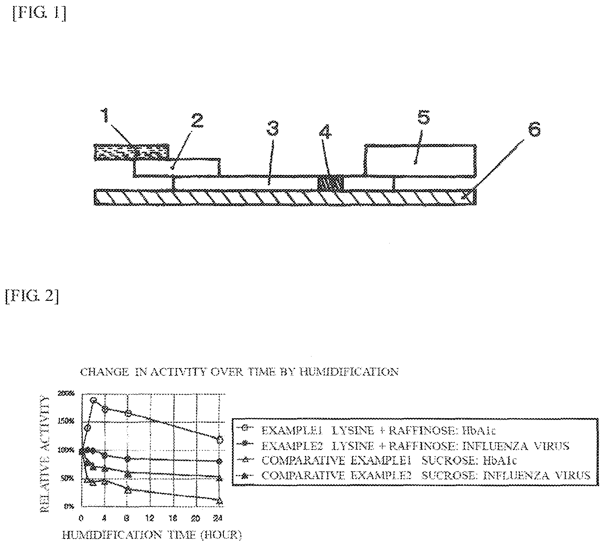

As one embodiment of the immunochromatographic device of the present invention, as shown in FIG. 1, the device is generally constituted by a sample addition part, a labeling substance retaining part, an absorption part, and a backing sheet other than the chromatographic medium and the detection part described above. The order in which the above-mentioned respective parts are provided is as follows: the sample addition part (1), the labeling substance retaining part (2), the chromatographic medium (also referred to as "chromatographic medium part") (3), the detection part (4), and the absorption part (5) in the order in which a sample is developed.

The sample addition part (1) is a part where a specimen sample is added in the immunochromatographic device. The sample addition part (1) can be constituted by a porous sheet having a property such that a sample is rapidly absorbed, but the sample promptly migrates therein. Examples of the porous sheet include a cellulose filter paper, glass fiber, polyurethane, polyacetate, cellulose acetate, nylon, and a cotton cloth.

The labeling substance retaining part (2) contains the below-mentioned labeling substance bound to an antibody which binds to a substance to be detected in advance. The substance to be detected is bound to the antibody and labeled with the antibody when the substance to be detected migrates in the labeling substance retaining part. The labeling substance retaining part (2) is composed of, for example, a glass fiber non-woven fabric, a cellulose film, or the like.

As the chromatographic medium (3) and the detection part (4), the above-mentioned chromatographic medium of the present invention is used.

The absorption part (5) is provided as needed at an end of the chromatographic medium part (3) for allowing the absorption part to absorb a liquid such as the specimen, the developing solution, etc. having passed through the detection part (4). In the immunochromatographic device of the present invention, as the absorption part (5), for example, a material in which a polymer such as an acrylic acid polymer and a hydrophilic agent having an ethylene oxide group or the like are included in glass fiber, pulp, cellulose fiber, or the like or a non-woven fabric thereof can be used. It is preferably glass fiber. By composing the absorption part (5) of glass fiber, the backward migration of the sample solution can be greatly reduced.

The backing sheet (6) is a base material. By applying an adhesive to one surface or sticking an adhesive tape to one surface, the surface has adhesiveness, and on the adhesive surface, part or all of the sample addition part (1), the labeling substance retaining part (2), the chromatographic medium part (3), and the absorption part (5) are provided in close contact with the surface. The backing sheet (6) is not particularly limited as the base material as long as it becomes impermeable to a sample solution and also is impermeable to moisture by the adhesive.

Further, in the present invention, by combining a specimen diluent and the above-mentioned immunochromatographic device, an immunochromatographic kit can be formed.

The specimen diluent can also be used as a developing solution, however, in general, water is used as a solvent, and thereto, a buffer solution, a salt, and a nonionic surfactant, and further, for example, one type or two or more types of a protein, a polymeric compound (PVP, etc.), an ionic surfactant or a polyanion, or an antimicrobial agent, a chelating agent, etc. for promoting an antigen-antibody reaction or suppressing a nonspecific reaction may be added.

In the case where it is used as a developing solution, a mixture obtained by mixing a specimen and the developing solution in advance can be supplied and added to the sample addition part to effect development, or a specimen is supplied and added to the sample addition part first, and thereafter, the developing solution may be supplied and added to the sample addition part to effect development.

In the immunochromatographic analysis method of the present invention, the following steps (1) to (4) are sequentially performed, and a substance to be detected contained in a specimen is detected using the above-mentioned immunochromatographic kit.
(1) a step of adding a specimen to the sample addition part along with a specimen diluent
(2) a step of allowing a labeling substance retained in the labeling substance retaining part to recognize the substance to be detected contained in the specimen
(3) a step of developing the specimen and the labeling substance in the chromatographic medium as a mobile phase
(4) a step of detecting the substance to be detected in the developed mobile phase in the detection part

Hereinafter, the respective steps will be described.

### (1) Step of adding specimen to sample addition part along with specimen diluent

In the step (1), in the first place, it is preferred to prepare a specimen-containing solution by adjusting or diluting a specimen with a specimen diluent to such a concentration that the specimen migrates smoothly in the chromatographic medium without decreasing the measurement accuracy. In the second place, the specimen-containing solution is added to the sample addition part (1) in a predetermined amount (generally from 0.1 to 2 mL). When the specimen-containing solution is added, the specimen-containing solution starts to migrate in the sample addition part (1).

### (2) Step of allowing labeling substance retained in labeling substance retaining part to recognize substance to be detected contained in specimen

The step (2) is a step in which the specimen-containing solution added to the sample addition part in the step (1) is migrated to the labeling substance retaining part (2), and the labeling substance retained in the labeling substance retaining part is allowed to recognize the substance to be detected in the specimen.

The labeling substance labels the antibody. In the labeling of a detection reagent in immunochromatography, in general, an enzyme or the like is also used, however, it is preferred to use an insoluble carrier as the labeling substance because it is suitable for visually determining the presence of a substance to be detected. The labeled detection reagent can be prepared by sensitizing an antibody to the insoluble carrier. A method for sensitizing the antibody to the insoluble carrier may be performed in accordance with a known method.

As the insoluble carrier to serve as the labeling substance, metal particles such as gold, silver, or platinum, metal oxide particles such as iron oxide, non-metal particles such as sulfur, latex particles composed of a synthetic polymer, or other insoluble carrier can be used.

The insoluble carrier is the labeling substance suitable for visually determining the presence of the substance to be detected, and is preferably a colored substance in order to facilitate the determination by visual observation. The metal particles and the metal oxide particles exhibit a specific natural color per se according to the particle diameter, and the color can be utilized as a label.

As the insoluble carrier to serve as the labeling substance, particularly, gold particles are preferred because detection is simple and easy, aggregation hardly occurs, and nonspecific color development is less likely to occur. The average particle diameter of the gold particles is, for example, from 10 nm to 250 nm, preferably from 35 nm to 120 nm. The average particle diameter can be obtained by randomly measuring the projected area equivalent circle diameters of 100 particles using a projected image taken by a transmission electron microscope (TEM, JEM-2010, manufactured by JEOL Ltd.), and calculating the average of the projected area equivalent circle diameters.

### (3) Step of developing specimen and labeling substance in chromatographic medium as mobile phase

The step (3) is a step in which after the substance to be detected is recognized by the labeling substance in the labeling substance retaining part in the step (2), the specimen and the labeling substance are allowed to pass on the chromatographic medium as a mobile phase.

### (4) Step of detecting substance to be detected in developed mobile phase in detection part

The step (4) is a step in which the substance to be detected in the specimen having passed on the chromatographic medium as the mobile phase is specifically reacted and bound so as to be interposed like a sandwich between the labeling reagent and the protein fixed in the detection part by a specific binding reaction with the protein which is the detection substance, and the detection part is colored.

In the case where the substance to be detected is not present, the labeling reagent dissolved in an aqueous component of the sample does not cause a specific binding reaction even if it passes through the detection part on the chromatographic medium and therefore, the detection part is not colored.

In the present invention, the detection part includes a tri- or higher polysaccharide and a basic amino acid, and therefore, the denaturation of a protein which is a detection target fixed in the detection part is sufficiently suppressed so as to sufficiently enhance the storage stability, and thus, the decrease in the activity of the detection substance can be prevented.

Finally, the aqueous component of the specimen-containing solution migrates to the absorption part (5).

### Examples

Hereinafter, the present invention will be further described by way of Examples and Comparative Examples, however, the present invention is not limited to the following Examples.

### [Example 1]

### 1. Preparation of immunochromatographic kit

### (1) Preparation of chromatographic medium and detection part

As a membrane to be used in the chromatographic medium, a sheet composed of nitrocellulose (manufactured by Millipore, Inc., trade name: HF 75, 300 mm x 25 mm) was used.

Subsequently, 150 µL of a solution obtained by diluting an anti-HbAlc monoclonal antibody (primary antibody) to a concentration of 1.0 mg/mL with a phosphate buffer solution (pH 7.4) containing 5 mass% isopropyl alcohol was applied to a detection part (detection line) with a width of 1 mm in a length of 300 mm on the dried membrane. Incidentally, as the solution, a solution having a lysine concentration of 8 mmol/mL (20 nmol per chromatographic medium) and a raffinose concentration of 10 mmol/mL (the concentration which gives 25 nmol per chromatographic medium) was prepared.

Further, in order to confirm the presence or absence of development of a gold nanoparticle labeling reagent or the developing rate, on the downstream of the detection part, a solution obtained by diluting a goat-derived antiserum having affinity in a wide range for a gold nanoparticle labeling substance, an animal meat protein which is the substance to be detected, and the like with a phosphate buffer solution (pH 7.4) was applied to a control region (control line). Thereafter, the solution was dried at 50°C for 30 minutes, and then dried at room temperature overnight, whereby a chromatographic medium and a detection part were prepared.

### (2) Preparation of sample addition part

As a sample addition part, a non-woven fabric composed of glass fiber (manufactured by Millipore, Inc., 300 mm × 30 mm) was used.

### (3) Preparation of labeling substance retaining part

To 0.5 mL of a colloidal gold suspension (manufactured by Tanaka Kikinzoku Kogyo K.K., LC 40 nm), 0.1 mL of a solution of an anti-HbAlc monoclonal antibody (secondary antibody) diluted to a concentration of 0.05 mg/mL with a phosphate buffer solution (pH 7.4) was added, and the resulting mixture was left to stand at room temperature for 10 minutes.

Subsequently, 0.1 mL of a phosphate buffer solution (pH 7.4) containing 1 mass% bovine serum albumin (BSA) was added thereto, and the resulting mixture was left to stand at room temperature for an additional 10 minutes. Thereafter, the mixture was thoroughly stirred, and then centrifuged at 8000 × g for 15 minutes. After removing the supernatant, 0.1 mL of a phosphate buffer solution (pH 7.4) containing 1 mass% BSA was added thereto. According to the above-mentioned procedure, a labeling substance solution was prepared.

A solution obtained by adding 300 µL of a 10 mass% aqueous solution of trehalose and 1.8 mL of distilled water to 300 µL of the above-prepared labeling substance solution was added uniformly to a 15 mm x 300 mm glass fiber pad (manufactured by Millipore, Inc.), followed by drying in a vacuum dryer, whereby a labeling substance retaining part was prepared.

### (4) Preparation of immunochromatographic device

Subsequently, an immunochromatographic device as shown in FIG. 1 was prepared using the above-prepared chromatographic medium, detection part, sample addition part, and labeling substance retaining part. To a base material composed of a backing sheet, the sample addition part, the labeling substance retaining part, the chromatographic medium having the detection part, and a non-woven fabric made of glass fiber as an absorption part for absorbing the developed sample and labeling substance were sequentially bonded. Then, the resulting material was cut to a width of 5 mm by a cutting machine, whereby an immunochromatographic device was prepared. The length in the direction of developing the sample in each of the labeling substance retaining part and an incubation part was set to 8 mm.

### (5) Specimen diluent

A 50 mM HEPES buffer solution (pH 7.5) containing 1 mass% of a nonionic surfactant (a 1:1 mixture of MN-811 (trade name) manufactured by NOF Corporation and NP-40 (trade name) manufactured by Nacalai Tesque, Inc.), 80 mM potassium chloride, 20 mM guanidine hydrochloride, and 0.4 wt% polyvinylpyrrolidone (average molecular weight: 360,000) was prepared and used as a reagent for performing a dilution treatment of a specimen.

### 2. Measurement

The above-prepared immunochromatographic device was placed in a humidifier and left in a state where the humidity was 95% and the temperature was 37°C for a time shown in Table 1. Thereafter, blood was collected by pricking the fingertip of each of healthy adult males and diabetic male patients, and each blood sample in which the concentrations of HbAO and HbAlc were measured by a latex agglutination method was mixed so that the concentration of HbAlc was 6.5%. The thus prepared HbAlc specimen was diluted to 100 times with the above-mentioned specimen diluent, and 150 µL of the thus prepared specimen sample was placed on the sample addition part of the immunochromatographic device and developed, and the degree of coloration (color development value) of the detection part was measured using a densitometer (manufactured by Hamamatsu Photonics K.K., hereinafter the same shall apply). The results are shown in Table 1 and FIG. 2. In the table, the relative activity is expressed as the ratio of the color development value when the color development value measured in the device which was not placed in the humidifier (the elapsed time was 0) was assumed to be 100%. Incidentally, the color development value was measured using a specimen (negative specimen) having a concentration of HbAl of 0% and composed only of HbAO, corresponding to each of Examples and Comparative Examples, and the measurement values were all less than 5 mAbs, and false-positive results were not confirmed.

### [Example 2]

Example 1 was repeated except that an "anti-influenza virus antibody" was used in place of the "anti-HbAlc monoclonal antibody" as the primary antibody and the secondary antibody and the specimen sample was changed to an influenza virus antigen. The degree of coloration (color development value) of the detection part was measured using a densitometer. The results are shown in Table 1 and FIG. 2.

### [Comparative Example 1]

Example 1 was repeated except that "sucrose" was used in place of "lysin and raffinose" in the detection part. Incidentally, in the preparation of the detection part, a solution having a sucrose concentration of 10 mmol/mL (the concentration which gives 20 nmol per chromatographic medium) was used, and the degree of coloration (color development value) of the detection part was measured using a densitometer. The results are shown in Table 2 and FIG. 2.

### [Comparative Example 2]

Example 2 was repeated except that "sucrose" was used in place of "lysin and raffinose" in the detection part. Incidentally, in the preparation of the detection part, a solution having a sucrose concentration of 10 mmol/mL (the concentration which gives 20 nmol per chromatographic medium) was used, and the degree of coloration (color development value) of the detection part was measured using a densitometer. The results are shown in Table 2 and FIG. 2.

### [Table 1]

**Table 1**

| | Substance to be detected | Detection part | | | | | | | |
|---|---|---|---|---|---|---|---|---|---|
| Example 1 | HbA1c | Lysine: 20 nmol Raffinose: 25 nmol | Elapsed time (hr) | 0 | 1 | 2 | 4 | 8 | 24 |
| | | | Color development value (mAbs) | 59.3 | 82.7 | 112.2 | 102.8 | 98.7 | 71.3 |
| | | | Relative activity (%) | 100 | 139 | 189 | 173 | 166 | 120 |
| Example 2 | Influenza virus | Lysine: 20 nmol Raffinose: 25 nmol | Elapsed time (hr) | 0 | 1 | 2 | 4 | 8 | 24 |
| | | | Color development value (mAbs) | 51.3 | 52.3 | 51.4 | 46.9 | 43.8 | 41.7 |
| | | | Relative activity (%) | 100 | 102 | 100 | 91 | 85 | 81 |

### [Table 2]

**Table 2**

| | Substance to be detected | Detection part | | | | | | | |
|---|---|---|---|---|---|---|---|---|---|
| Comparative Example 1 | HbA1c | Sucrose: 20 nmol | Elapsed time (hr) | 0 | 1 | 2 | 4 | 6 | 24 |
| | | | Color development value (mAbs) | 191.8 | 96.8 | 85.7 | 91.0 | 62.5 | 26.6 |
| | | | Relative activity (%) | 100 | 50 | 45 | 47 | 33 | 14 |
| Comparative Example 2 | Influenza virus | Sucrose: 20 nmol | Elapsed time (hr) | 0 | 1 | 2 | 4 | 6 | 24 |
| | | | Color development value (mAbs) | 49.9 | 41.0 | 36.6 | 35.2 | 30.9 | 26.6 |
| | | | Relative activity (%) | 100 | 82 | 73 | 71 | 62 | 53 |

In Table 1 and FIG. 2, in Examples 1 and 2, the detection part includes lysine which is a basic amino acid and raffinose which is a trisaccharide, and therefore, the decrease in the activity of the detection substance was suppressed when it was left in an environment where the humidity was 95% and the temperature was 37°C for a certain period of time. Further, in Example 1 in which HbAlc was used as the substance to be detected, the result that the activity of the detection substance was improved was obtained.

On the other hand, in Table 2 and FIG. 2, in Comparative Examples 1 and 2 in which only sucrose which is a disaccharide was included, the activity of the detection substance was decreased.

### [Example 3]

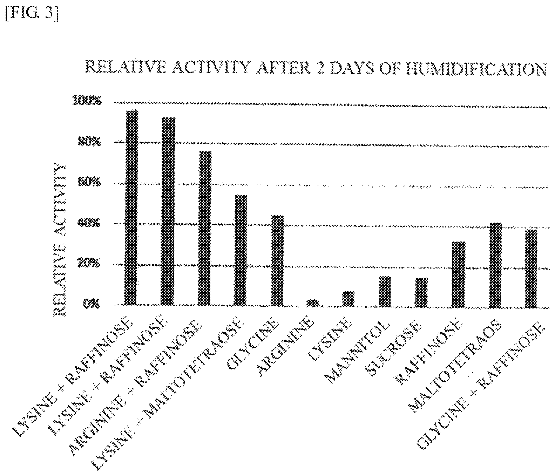

The immunochromatographic device used in Example 1 was left for 2 days in a humidified environment where the temperature was from 24 to 26°C and the humidity was from 40 to 70%. As the specimen, HbAlc which is the same as in Example 1 was used, and the degree of coloration (color development value) of the detection part in the immunochromatographic device before being left and the degree of coloration (color development value) of the detection part in the immunochromatographic device after being left for 2 days were measured using a densitometer, respectively. The results are shown in Table 3 and FIG 3. In Table 3, the relative activity is expressed as the ratio of the color development value measured in the immunochromatographic device after being left for 2 days when the color development value measured in the immunochromatographic device before being left was assumed to be 100%.

### [Example 4]

Example 3 was repeated except that the amounts of lysine and raffinose applied to the detection part of the immunochromatographic device in Example 3 were changed as shown in Table 3. The results are shown in Table 3 and FIG. 3.

### [Example 5]

Example 3 was repeated except that arginine and raffinose were applied in amounts shown in Table 3 to the detection part of the immunochromatographic device in Example 3. The results are shown in Table 3 and FIG. 3.

### [Example 6]

Example 3 was repeated except that lysine and maltotetraose were applied in amounts shown in Table 3 to the detection part of the immunochromatographic device in Example 3. The results are shown in Table 3 and FIG. 3.

### [Comparative Example 3]

Example 3 was repeated except that glycine was applied in an amount shown in Table 3 to the detection part of the immunochromatographic device in Example 3. The results are shown in Table 3 and FIG. 3.

### [Comparative Example 4]

Example 3 was repeated except that arginine was applied in an amount shown in Table 3 to the detection part of the immunochromatographic device in Example 3. The results are shown in Table 3 and FIG. 3.

### [Comparative Example 5]

Example 3 was repeated except that lysine was applied in an amount shown in Table 3 to the detection part of the immunochromatographic device in Example 3. The results are shown in Table 3 and FIG. 3.

### [Comparative Example 6]

Example 3 was repeated except that mannitol was applied in an amount shown in Table 3 to the detection part of the immunochromatographic device in Example 3. The results are shown in Table 3 and FIG. 3.

### [Comparative Example 7]

Example 3 was repeated except that sucrose was applied in an amount shown in Table 3 to the detection part of the immunochromatographic device in Example 3. The results are shown in Table 3 and FIG. 3.

### [Comparative Example 8]

Example 3 was repeated except that raffinose was applied in an amount shown in Table 3 to the detection part of the immunochromatographic device in Example 3. The results are shown in Table 3 and FIG. 3.

### [Comparative Example 9]

Example 3 was repeated except that maltotetraose was applied in an amount shown in Table 3 to the detection part of the immunochromatographic device in Example 3. The results are shown in Table 3 and FIG. 3.

### [Comparative Example 10]

Example 3 was repeated except that glycine and raffinose were applied in amounts shown in Table 3 to the detection part of the immunochromatographic device in Example 3. The results are shown in Table 3 and FIG. 3.

### [Table 3]

**Table 3**

| | Detection part | Color development value (mAbs) | | Relative activity (%) |
|---|---|---|---|---|
| | | Humidification time | | |
| | | on Day 0 | on Day 2 | |
| Example 3 | Lysine: 20 nmol Raffinose: 25 nmol | 59.3 | 56.6 | 95 |
| Example 4 | Lysine: 30 nmol Raffinose: 20 nmol | 55.4 | 51.1 | 92 |
| Example 5 | Arginine: 15 nmol Raffinose: 30 nmol | 72.5 | 55.2 | 76 |
| Example 6 | Lysine: 20 nmol Maltotetraose: 25 nmol | 56.6 | 31 | 55 |
| Comparative Example 3 | Glycine: 20 nmol | 40.2 | 18.1 | 45 |
| Comparative Example 4 | Arginine: 20 nmol | 122.7 | 4 | 3 |
| Comparative Example 5 | Lysine: 20 nmol | 110.7 | 8.2 | 7 |
| Comparative Example 6 | Mannitol: 20 nmol | 78.7 | 12 | 15 |
| Comparative Example 7 | Sucrose: 20 nmol | 191.8 | 27.8 | 14 |
| Comparative Example 8 | Raffinose: 20 nmol | 89 | 29.3 | 33 |
| Comparative Example 9 | Maltotetraose: 20 nmol | 54.2 | 22.7 | 42 |
| Comparative Example 10 | Glycine: 20 nmol Raffinose: 25 nmol | 32.3 | 12.5 | 39 |

In Table 3 and FIG. 3, in Examples 3 to 6, the detection part includes lysine or arginine which is a basic amino acid and a tri- or higher polysaccharide (raffinose or maltotetraose), and therefore, the decrease in the activity of the detection substance when the device was left for 2 days was suppressed. In particular, in Examples 3 and 4 in which the detection part includes both lysin and raffinose, the decrease in the activity of the detection substance was greatly suppressed.

On the other hand, in Comparative Example 3 in which only glycine which is a neutral amino acid is included, in Comparative Examples 4 and 5 in which only arginine or lysine which is a basic amino acid is included, in Comparative Examples 6 and 7 in which only mannitol which is a monosaccharide or sucrose which is a disaccharide is included, and in Comparative Examples 8 and 9 in which only raffinose which is a trisaccharide or maltotetraose which is a tetrasaccharide is included, the activity of the detection substance was remarkably decreased. Further, also in Comparative Example 10 in which glycine which is a neutral amino acid and raffinose which is a trisaccharide are included, the activity of the detection substance was remarkably decreased.

From the above results, it was found that by including both a basic amino acid and a tri- or higher polysaccharide in the detection part, the decrease in the activity of the detection substance is greatly suppressed.

While the present invention has been described in detail with reference to specific embodiments, it is apparent to those skilled in the art that various changes and modifications can be made without departing from the spirit and scope of the present invention. The present application is based on Japanese Patent Application (Japanese Patent Application No. 2016-010795) filed on January 22, 2016 and the entire contents of which are incorporated herein by reference.

### Reference Signs List

1. sample addition part
2. labeling substance retaining part
3. chromatographic medium
4. detection part
5. absorption part
6. backing sheet

## Claims

1. A chromatographic medium which is used in immunochromatography for detecting a substance to be detected in a specimen, comprising:
a detection part in which a detection substance composed of a protein is fixed,
wherein the detection part includes a tri- or higher polysaccharide and a basic amino acid.

2. The chromatographic medium according to claim 1,
wherein the polysaccharide included in the detection part is at least one polysaccharide selected from the group consisting of raffinose, nigerotriose, maltotriose, melezitose, maltotriulose, kestose, nystose, nigerotetraose, stachyose, and maltotetraose.

3. The chromatographic medium according to claim 1 or 2,
wherein the detection part includes the polysaccharide in an amount of 5 to 50 nmol.

4. The chromatographic medium according to any one of claims 1 to 3,
wherein the detection part includes the basic amino acid in an amount of 5 to 50 nmol.

5. The chromatographic medium according to any one of claims 1 to 4,
wherein the molar ratio of the polysaccharide to the basic amino acid included in the detection part is from 1:10 to 10:1.

6. The chromatographic medium according to any one of claims 1 to 5,
wherein the detection part includes at least one basic amino acid selected from the group consisting of arginine, lysine, ornithine, and histidine.

7. The chromatographic medium according to any one of claims 1 to 6,
wherein the detection part includes lysine.

8. The chromatographic medium according to any one of claims 1 to 7,
wherein the substance to be detected is a glycoprotein.

9. The chromatographic medium according to claim 8,
wherein the glycoprotein is HbA1c.

10. An immunochromatographic device, comprising:
the chromatographic medium according to any one of claims 1 to 9.

11. An immunochromatographic kit, comprising:
the immunochromatographic device according to claim 10 and
a specimen diluent.

12. An immunochromatographic analysis method, wherein the following steps (1) to (4) are sequentially performed using the immunochromatographic kit according to claim 11:
(1) a step of adding a specimen to a sample addition part along with a specimen diluent,
(2) a step of allowing a labeling substance retained in a labeling substance retaining part to recognize a substance to be detected contained in the specimen,
(3) a step of developing the specimen and the labeling substance in a chromatographic medium as a mobile phase; and
(4) a step of detecting the substance to be detected in the developed mobile phase in a detection part.
